# EUROPEAN PATENT APPLICATION

(11) **EP 2 514 385 A1**
(43) Date of publication of application: **24.10.2012**
(21) Application number: 12166680.4
(22) Date of filing: 03.04.2009
(51) Int. Cl.: A61B 18/28, A61B 17/30, A61B 17/00

(54) **Tissue treatment device and method**

(30) Priority: 11.04.2008 US 44385 P; 02.04.2009 US 417139
(62) Divisional of application: 09004971.9
(71) Applicant: Lumenis Ltd., 20692 Yokneam (IL)
(72) Inventor: Lewinsky, Reuven, 36005 Alonei Aba (IL); Khen, Roee, 34990 Haifa (IL); Mintz, Yoav, 96410 Jerusalem (IL)
(74) Representative: Critten, Matthew Peter

(57) **Abstract**

Disclosed is a treatment device. The treatment device includes a treatment tip that includes one or more members with contacting surfaces to contact a target area, the tip being mechanically coupled to a body member. The tip also includes one or more heating elements placed on at least one of the one or more members of the treatment tip, the one or more heating elements configured to be heated by laser radiation and to direct heat to treat at least a portion of the target area.

## Description

Tools and devices to perform surgical operations through remote manipulation of the tools/devices (e.g., controlling an applicator or treatment tip using a user interface located at a distance away from the actual applicator or tip, or a handle with controls on the outer side of the patient's body) include, for example, laparoscopic surgery tools in which a viewing apparatus (laparoscope) is inserted through a small incision below, for example, the umbilicus. The laparoscope has a working channel through which various surgical tools, e.g., a laser adaptor enabling to fire a direct laser beam or coupled to an optical waveguide (such as a fiber), to irradiate a target area, are passed. The tools passed through the scope's working channel include tools to perform surgical operations that include, for example cutting, ablation, coagulation, suturing, etc. Under some circumstances, additional tools may be inserted through side holes to enable access to organs from different angles, thus providing more degrees of freedom and flexibility.

Laparoscopic tools may perform mechanical manipulations (grasping, cutting, etc.) which can be combined with the delivery of energy (e.g., RF, Ultrasound) to, for example, coagulate blood vessels contained in the tissue being cut. Conventional instruments/devices that apply energies to target tissue, or to some other type of target areas, frequently cause significant collateral thermal/radiation damage to adjacent tissues. Conventional tools that apply ultrasound energy at, for example, 50 KHz (e.g., the Harmonic Scalpel manufactured by Ethicon Endosurgery) are relatively safe and effective, but often lack flexibility. Flexibility (e.g., the ability to turn the tip of the tool in various directions) is important, especially when a single puncture approach (i.e., entering the body through a single penetration site) is adopted.

Conventional flexible tip laparoscopic tools include mechanisms to control the tip orientation/position and a lever to open and close the jaws. The jaws can be used to hold the tissue, suture using a needle, tear the tissue etc. Such flexible tip laparoscopic tools, however, generally include energy delivery mechanisms such as RF which only enable limited safe and effective substantially concomitant performance of cutting and coagulation operations.

### SUMMARY

The present disclosure relates to a treatment devices and methods.

In one aspect, a treatment device is disclosed. The treatment device includes a treatment tip that includes one or more members with contacting surfaces to contact a target area, the tip being mechanically coupled to a body member. The tip also includes one or more heating elements placed on at least one of the one or more members of the treatment tip, the one or more heating elements configured to be heated by laser radiation and to direct heat to treat at least a portion of the target area.

Embodiments of the treatment device may include one or more of the following features.

The device may further include the body member.

The target area includes tissue of a patient.

The device may further include at least one laser fiber having a distal end positioned proximate to the one or more heating elements, the at least one laser fiber configured to emit radiation substantially directed at the one or more heating elements.

The device may further include a thermal insulation layer disposed proximate the one or more heating elements to thermally isolate the one or more heating elements from other areas of the treatment tip.

The treatment tip may include at least one mechanically movable member. The at least one mechanically moveable member may be rotateable about a longitudinal axis of the body member. The at least one mechanically moveable member may include at least one closeable jaw to hold a section of the target area, the at least one closeable jaw including at least one opposite facing contacting surface such that upon actuation of the at least one closeable jaw the at least one opposite facing contacting surface is displaced.

The heating elements are configured to direct heat to the at least the portion of the area to be treated to cause one or more of, for example, cutting a section of tissue, ablating the section of the tissue and/or coagulating a blood vessel within the section of the tissue.

The device may further include at least one sensor to measure temperature of at least one of the one or more heating elements, and a controller to regulate operations of the one or more heating elements based on the measured temperature of the at least one of the one or more heating elements.

The one or more heating elements may include two heating elements arranged on one of the one or more members in a substantially parallel orientation such that application of heat to the at least the portion of the target area to be treated causes the formation of two coagulation sites on the target area. The device may further include a cutting instrument disposed between the two heating elements.

In another aspect, a method is disclosed. The method includes placing proximate a target area a treatment device having a treatment tip including one or more heating elements. The method further includes heating with laser radiation the one or more heating elements, and directing heat from the one of the one or more heating elements to treat at least a portion of the target area.

Embodiments of method may include any of the features described above in relation to the device, as well one or more of the following features.

The target area may include tissue of a patient.

Heating with laser radiation the one or more heating elements may include directing emitted laser radiation at the one or more heating elements from a distal end of a laser fiber positioned proximate to the one or more heating elements.

The method may further include measuring the temperature of at least one of the one or more heating elements, and regulating the operation of the one or more heating elements based on the measured temperature of the at least one of the one or more heating elements.

In a further aspect, a treatment apparatus is disclosed. The apparatus includes an endoscope containing a flexible body in a working channel of the endoscope, and a treatment tool having a treatment tip including one or more heating elements configured to be heated by laser radiation and to direct heat to treat at least a portion of a target area, the treatment tool coupled to a body member coupled to the flexible body.

Embodiments of apparatus may include any of the features described above in relation to the device and in relation to the method, as well one or more of the following features.

The apparatus may further include at least one actuation mechanism to control movement of the treatment tool. At least a part of the at least one actuation mechanism may be disposed within the inner channel of the flexible body.

The body member may include an extendible body member passing through an inner channel of the flexible body.

The apparatus may further include at least one laser fiber having a distal end positioned proximate to the one or more heating elements, the laser fiber configured to emit radiation substantially directed at the one or more heating elements.

The treatment tip may include at least one closeable jaw to hold a section of the target area, the at least one closeable jaw including at least one opposite facing contacting surface such that upon actuation of the at least one closeable jaw the at least one opposite facing contacting surface is displaced.

Details of one or more implementations are set forth in the accompanying drawings and in the description below. Further features, aspects, and advantages will become apparent from the description, the drawings, and the claims.

### BRIEF DESCRIPTION OF THE DRAWINGS

FIG. 1 is diagram of a treatment device with a treatment tip.
FIG. 2 is a cross-sectional diagram of the treatment tip shown in FIG. 1.
FIGS. 3A-B are diagrams providing enlarged views of closeable jaws in the jaws' open position.
FIG. 4 is a diagram providing an enlarged view of closeable jaws in a closed position.
FIG. 5A is a schematic diagram of a scope-based apparatus that includes one or more treatment tips.
FIG. 5B is a schematic diagram of a scope-based apparatus that includes a flexible treatment tip with an extendible body member.
FIG. 5C is a schematic diagram of a treatment tip coupleable to a scope-based apparatus.
FIG. 6 is a flow chart of a procedure to treat a target area.

### DETAILED DESCRIPTION

Disclosed are devices, apparatuses and methods in which laser energy is used to heat heating elements (e.g., tissue heating elements) placed on at least one member of a treatment tip used to perform operations on a target area. In some embodiments, the at least one treatment tip (e.g., closeable jaws) is coupled to a flexible laparoscopic tool. The laser-heated elements of the treatment tip may be heated to temperatures suitable to perform such operations as cutting, coagulating blood vessels in the tissue being treated, etc. Heating of the tissue heating elements is performed, for example, by irradiating the tissue heating elements with radiation (e.g., laser radiation) delivered by a suitable waveguide, such as a laser or optical fiber. The tissue heating elements can achieve high temperatures in a very short period of time. In some embodiments, a treatment tip that includes laser-heated tissue heating elements is used to perform other functions on different types of target areas. For example, the devices and methods described herein may be used in industrial-type-applications to perform operations on fabrics, materials, etc.

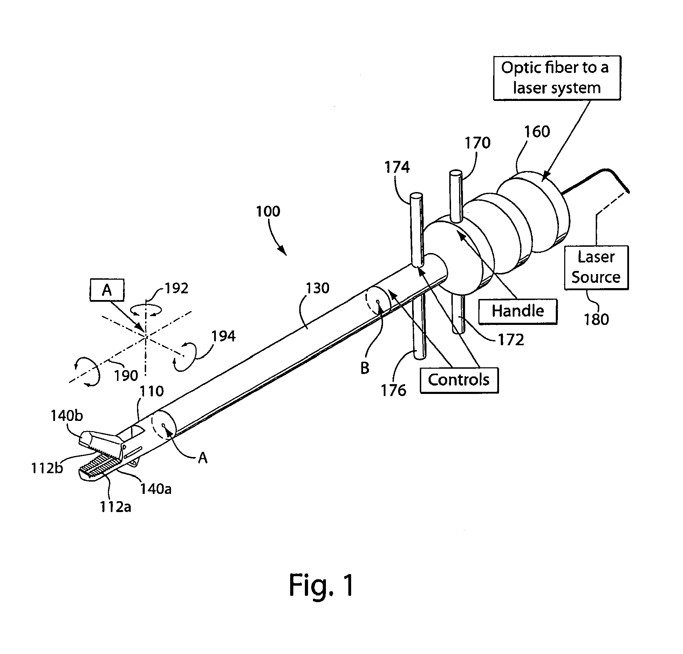

Referring to FIG. 1, a perspective view of a device 100 is shown. While the depicted device is configured to be used in surgeries, the device, or similar implementations thereof, may be used for other types of applications.

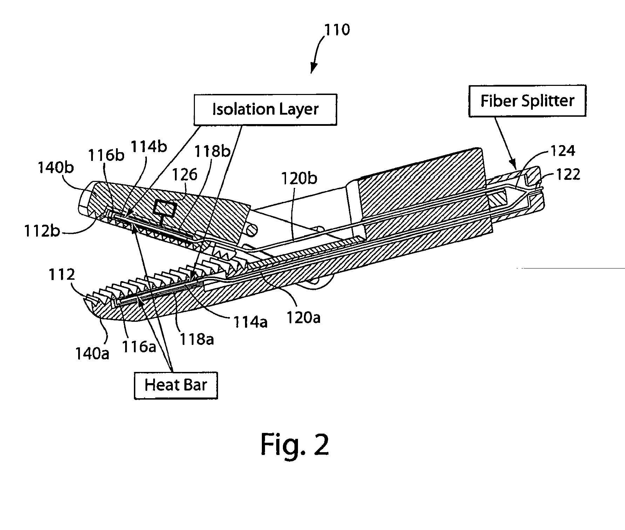

The device 100 includes a treatment tip 110 configured to perform one or more functions on the target area. The treatment tip is mechanically coupled to a body member 130 (e.g., an elongated shaft that includes part or all of at least one actuation mechanism to actuate and control operations of the treatment tip). As will become apparent, suitable body members also include flexible chords or tubes, which may be extendible (e.g., can be advanced and/or retracted as desired). As shown, in some embodiments, the treatment tip includes one or more members (e.g., closeable jaws) having contacting surfaces 112a and 112b to contact the target area and facilitate performance of the implemented operations on the target area. The contacting surfaces 112a and 112b may have an irregular structure (e.g., a roughened or jugged surface) to enable improved traction with the target area that is to come in contact with the contacting surfaces. Embedded in at least one of the contacting surfaces 112a and/or 112b of the closeable jaws 140a and 140b of the treatment tip 110 are one or more heating elements, such as heating elements 114a and 114b depicted in FIG. 2 (when the treatment tip is used to operate on tissue, the heating elements are sometimes referred to as tissue-heating elements). The heating elements are configured to transfer thermal energy to at least a portion of the target area being operated upon by the device 100.

With reference to FIG. 2, showing a cross-sectional diagram of the treatment tip 110, in some embodiments, the heating elements 114a and 114b are disposed in cavities 116a and 116b defined in the interior of the members (e.g., the arms or jaws 140a and 140b) of the treatment tip 100. The heating elements may be constructed from thermally conducting materials, such as metals, that transfer, e.g., through thermal dissipation, heat. In some embodiments, thermal isolation (insulation) layers 118a and 118b, e.g., air, ceramic materials, various polymers, etc., are lined along the inner and side walls defining the cavities 116a and 116b such that heat dissipating from the tissue heating elements 114a and 114b is directed to the outer surfaces of the elements that are placed in the members 140a and/or 140b, but does not permeate to other parts of the treatment tip 110, or to the body member 130 of the device 100. In some embodiments, the members 140a and/or 140b, including their respective contacting surfaces 112a and 112b, are constructed from non-heat conductive materials to prevent generated heat from being directed to the members 140a and 140b (including preventing heat being directed to the contacting surfaces 112a and 112b). Thus, substantially most of the heat generated in the heating elements 114a and 114b is conducted to one of the surfaces of the heating elements (i.e., the surface facing the same direction as the surface of the contacting surfaces 112a and 112b), and from there are directed to the target tissue substantially in contact with the outer surfaces of the heating elements.

Positioned proximate to the ends of the heating elements 114a and 114b are the emitting ends of radiation waveguides 120a and 120b. Such radiation waveguide include optical fibers (e.g., laser fibers) that enable light radiation, e.g., laser radiation or non-coherent radiation (such as Intense Pulse Light radiation) to be directed from a light source (not shown in the figures) via the fibers 120a and 120b and to be emitted at the tissue heating elements 114a and 114b (or any other heating element). Particularly, in some embodiments, a laser source (e.g., a laser source 180 schematically shown in FIG. 1) is used to provide the energy required to enable the tissue heating elements to reach significantly high temperatures at a rate significantly faster than any conventional surgical tool (e.g., in some embodiments, reach a temperature of, for example, up to 250° C within a relative short period of time, e.g., about 2 seconds using a laser device having 5-20 W power). To heat the tissue heating elements, different types of lasers may be used, including an Nd:YAG laser, a Ho:YAG laser, a CO₂ laser, an Er:YAG laser, a KTP device, laser diodes, etc. Radiation generated by the radiation source may initially be delivered through a single flexible coated fiber 122 (a portion of which is shown in FIG. 2) passing through an inner channel defined in the body member 130. Along the way, either as part of the reusable device or alternatively as part of a single use tool, a splitter 124 is employed to transfer the energy into the two separate fibers 120a and 120b, each disposed in a channel within the treatment tip 110 culminating with the respective heating elements 114a and 114b. Radiation emitted through the ends of the fibers 120a and 120b is thus applied to the heating elements 114a and 114b to heat them. The splitter 124 may be implemented in various ways and positioned at any location along the path defined from the laser source to the locations of the heating elements in the treatment tip.

The tissue heating elements 114a and 114b are of such shape and size to enable the elements to quickly absorb energy and have their temperature increased upon being irradiated with laser radiation. In some embodiments, the heating elements 114a and 114b include bores extending along at least a portion of each of the heating elements to receive substantially the entire radiation emitted from the tips of the laser fibers 120a and 120b, thus expediting the heating of the heating elements.

In some embodiments, a temperature sensor 126 may be thermally coupled to at least one of the heating elements (e.g., the heating elements 114a and/or 114b) or to a specific location on the closeable jaws 140a or 140b to measure the temperature. Based on the measured temperature, the level of radiation applied to the heating elements 114a and 114b via the energy transmitting fibers (e.g., the fibers 122, 120a and 120b) may be controlled or regulated. If a particular temperature is required (e.g., a temperature specified by an operator of the device 100), a controller receiving from the sensor 126 data representative of the temperature of the at least one heating element determines whether and/or at what level to transfer radiation to the heating element. For example, if it is determined that the specified temperature required for a particular operation (e.g., coagulation of blood vessels) has not yet been reached, the controller will cause continued delivery of radiation from the radiation source to the heating elements 114a and 114b. When the measured temperature substantially equals the specified temperature required for the particular operation, the controller may cause delivery of the radiation to the heating element to be suspended (e.g., terminate generation of radiation). The controller may also use the data provided by the temperature sensor to, for example, maintain the heating elements at a desired temperature for the duration of a required operation (e.g., the controller may act as a thermostat). For example, the controller may regulate the radiation source to produce radiation that will result in the desired temperature being maintained. Deviations from the desired temperature, as indicated by the measurements of the sensor, may cause the controller to alter one or more attributes relating to the radiation delivery (e.g., the energy of the radiation source) so that the temperature of the one or more heating elements 114a and/or 114b converges to the desired temperature. Thus, in such embodiments, the controller implements a closed-loop control mechanism using thermocouple(s), or other sensing elements, to prevent overheating of the heating elements and tissue. While an operator may press the trigger on the handle of the tool or the foot switch in a continuous manner, the controller may be configured to ensure that the temperature is maintained either at a specific temperature, or in a range of temperatures, required to achieve the desired effect without a significant heat build-up in the treatment tip.

In some embodiments, the controller may be implemented using a processor-based device (not shown). Such a processor-based device can receive control input data (e.g., temperature data) from, for example, a temperature sensor and/or through a user-interface, and, based on the input data, generate control signals to control the operation of the device (e.g., regulate application of radiation on the heating elements 114a and 114b). The processor-based device may include a computer and/or other types of processor-based devices suitable for multiple applications. Such devices can include volatile and non-volatile memory elements, and peripheral devices to enable input/output functionality. Such peripheral devices include, for example, a CD-ROM drive and/or flash drive, or a network connection, for downloading related content. Such peripheral devices may also be used for downloading software containing computer instructions to enable general operation of the controller, and for downloading software implemented programs to perform operations to control, for example, application of radiation to the heating elements of the device 100.

As further shown in FIGS. 1 and 2, the treatment tip may be implemented to include closeable jaws, particularly, the lower jaw 140a and the upper jaw 140b, that respectively include the contacting surfaces 112a and 112b. In such embodiments, the treatment tip 110 may be configured to perform pinching and/or holding operations to, for example, grab a section of the target area. When the section of the target area is so grabbed, heat generated by the tissue heating elements (e.g., heat generated through irradiation of the heating elements with laser radiation) is transferred to a portion of the tissue held by the closeable jaws, i.e., the portion substantially in contact with the outer surfaces of the heating elements 114a and 114b.

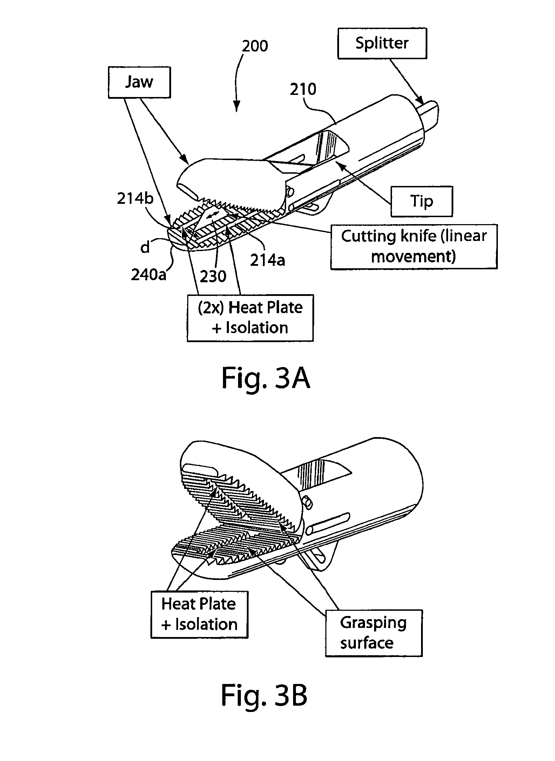

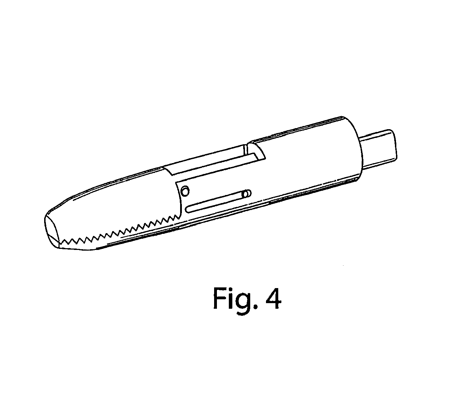

With reference to FIGS. 3A and 3B, enlarged views of closeable jaws in their open positions are shown. FIG. 4 is an enlarged view of closeable jaws, such as those depicted in FIGS. 3A-B, in the jaws' closed position.

In FIG. 3A, a treatment tip 210 of a device 200 includes two heating elements 214a and 214b that are arranged in a parallel orientation relative to each other on a member 240a (e.g., a lower closeable jaw) of the tip 210. As with the tissue heating elements 114a and 114b of the device 100, in some embodiments, the heating element 214a and 214b may be bars constructed from thermal conductive materials disposed in cavities defined in the member 240a. The heating elements 214a and 214b are heated, for example, using laser radiation directed at the heating elements (and delivered, for example, via laser fibers) to heat the elements 214a and 214b. Use of laser radiation enables heating the elements 214a and 214b to, in some embodiments, up to 250° C, in approximately 2 seconds.

As further shown in FIG. 3A, the heating elements on the lower member 240a are separated by a distance d to enable a concomitant treatment of the target area treated by the device 200. Optionally, a similar configuration may be implemented in the upper member 240b such that a total of four (4) tissue heating elements are included in the treatment tip. Thus, when applied to a tissue of a patient during surgery, the pair of parallel tissue heating elements 214a and 214b cause heat to be applied concomitantly at two locations. The heat applied can cause, in some embodiments, blood vessels at those two locations to coagulate. Once two lines of coagulation are formed on the tissue, the section of the target area between the two coagulated sections can be cut. Cutting of the target area between the two sections treated by the treatment tips can be performed using a cutting tool or applicator disposed between the heating elements, or can be performed by a separate tool not otherwise mechanically coupled to the device. For example, the cutting tool may include a mechanical blade, such as the blade 230 shown in FIG. 3A. In some embodiments, the cutting tool may include a laser device, or some other cutting tool.

In some embodiments, the closeable jaws include actuation mechanisms to control rotation and displacement of the closeable jaws. For example, the actuation mechanisms may include mechanisms to enable displacement of the upper and lower jaws relative to each other to open and close the jaws. Referring again to FIG. 1, as shown, the actuation mechanisms to control the motion of the treatment tip 110 (whether the treatment tip 110 includes a closeable jaws and/or other tools) includes actuation mechanisms to rotate the treatment tip at various orientations, for example, to rotate the treatment tip 110 about one or more axes of rotation. More particularly, in some embodiments, the treatment tip 110 is configured to rotate or pivot about three separate axes. For example, the treatment tip may be rotated about an axis 190 which is parallel to the longitudinal axis of the device 100. In some embodiments, the actuation mechanisms may include an actuation mechanism to pivot of the treatment tip 110 about the axis 192 (the axis 192, depicted in FIG. 1 as being substantially perpendicular to the axis 190 and substantially vertical). Thus, the treatment tip 110 may be controlled to move laterally relative to the longitudinal axis of the device 100. In some embodiments, the treatment tip 110 may also be actuated to cause the treatment tip to pivot about the axis 194 (the axis 194 is depicted as being substantially perpendicular to the axis 190 and the axis 192). Thus, the treatment tip can be controlled to be moved "up" and "down" (as illustrated in FIG. 1). Other actuation mechanisms to control movement of the treatment tip to cause it to move in other directions or orientations relative to the body 130 member (e.g., rotate or pivot about other axes) may be used. The various actuation mechanisms may act separately or simultaneously, resulting in a general spatial movement which represents the relative contribution of each vector component to the overall movement. This can be done by electrical, mechanical and/or as electro-mechanical mechanisms. In some embodiments, the treatment tip 110 including the one or more heating elements 114a and 114b may be coupled to a rigid body member 130 where the articulation point A shown in FIG. 1 does not exist, and as a result spatial movement of the treatment tip 110 about the axes 192 and 194 cannot be performed. Under those circumstances, movement of the tip 110 can be performed only with respect to the axis 190. In some embodiments, the treatment tip 110 is coupled to a flexible body member, in which case both the articulation points A and B constitute part of the device 100, and thus the treatment tip 110 can be moved about the axes 190, 192 and 194.

Control of the various actuation mechanisms that control the movement of the treatment tip 110 may be effectuated through a user interface that includes, for example, levers, buttons, knobs, and other types of user controllable elements. Such user-controllable elements may be arranged on the device 100. In response to user manipulation of the user controllable elements, the actuation mechanisms controlled via the one or more user-controllable elements are actuated, thus causing an associated movement or manipulation of the treatment tip. As shown in FIG. 1, the user controllable elements include lever 170, 172, 174 and 176, each of which may control and actuate one or more actuation mechanisms to which the levers are coupled to thus cause the treatment tip to be moved in some particular way. Another user-controllable interfacing element could be an ON-OFF button (not shown in FIG. 1) which controls application of laser radiation to the heating elements 114a and 114b to heat them at the operator's discretion. Another control button can alternate between cutting and coagulation modes, each of which is characterized by specific set of heating parameters.

It is to be noted that the device(s) described herein may be used without heating the heating elements. Thus, the device(s) can be used to perform its other functionalities, such as pinching (or grasping), cutting, etc., without directing heat to the target area. In that respect, the devices may be implemented to have multiple modes of operation. In some embodiments, some or all of the heating elements can be selectively heated. For example, only one of two opposing heating elements (i.e., heating elements placed on opposing members of a treatment tip) may be selected to be heated.

Alternatively and/or additionally, in some embodiments, a device with multiple treatment tips (e.g., multiple treatment tips that each includes closeable jaws) mechanically coupled to the device body may be used. An example of such a device with multiple treatment tips is a device including two pairs of closeable jaws (i.e., a device with two closeable-jaws-based treatment tips). Such a device could be used, for example, as a split tipped flexible laparoscopic tool. In embodiments that include a device with two pairs of closeable jaws, each pair of closeable jaws may be implemented in a manner similar to the implementation of the closeable-jaws-based treatment tips 110 and/or 210 respectively depicted in FIGS. 1 and 3A. Multiple tips can be arranged in various configurations relative to each other. For example, where the device includes two pairs of closeable jaws, the pairs can be arranged parallel to each other and be aligned on a common plane. Within the spatial distance separating the two parallel treatment tips with closeable jaws (also referred to as clamping jaws), for example, at the midpoint, a cutting tool may be disposed so that the cutting tool is also parallel and lies substantially on the same plane of the treatment tips. In some embodiments, one or more of the treatment tips may all be coupled to the energy source used to, for example, heat the heating elements on a particular treatment tip. The one or more treatment tips may each be controlled (e.g., activated) independently. For example, in some embodiments some of the one or more treatment tips may be operated at a particular instance of time, while the other treatment tips are inactive, and subsequently, the other treatment tips are activated while operation of the first activated tips is suspended.

In some embodiments, a flexible endoscope (e.g., bronchoscope, urethroscope, etc.) may be used for the treatment procedures, e.g., a laparoscopic procedure. An endoscope is a viewing tool which is usually passed through a natural orifice of the body (mouth, nose, rectum, vagina, urethra), or through an incision made on the body, to view the inside of the body. For example, an endoscope may be passed through an incision in the abdominal wall. Subsequent to the insertion of the flexible endoscope, a device that includes one or more treatment tips similar to the treatment tip 110 depicted in FIGS. 1-4 may be passed through the interior channel (also referred to as the working channel) of the endoscope. Thus, in such embodiments, the one or more treatment tips are coupled to a flexible body of the endoscope. The flexible body of the endoscope thus serves as a housing to provide a structural support to the one or more treatment tips passing through the endoscope.

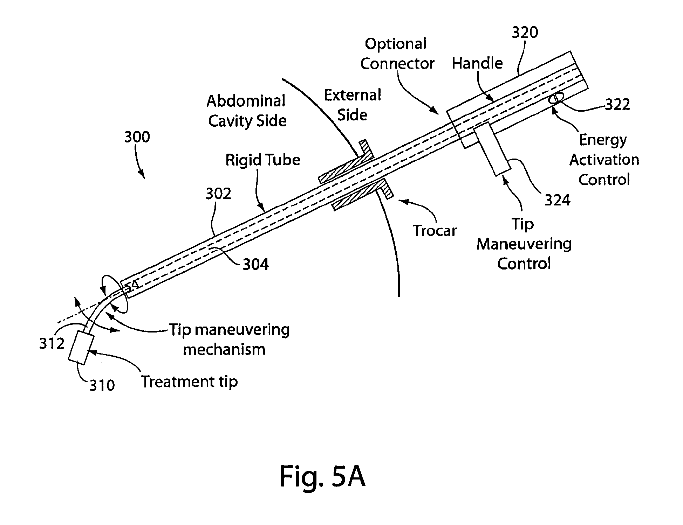

Thus, with reference to FIG. 5A, a schematic diagram of an apparatus 300 that includes one or more treatment tips 310 is shown. In the illustrated example, the apparatus 300 is used for surgical procedures, e.g., procedures within the abdominal cavity. In some embodiments, the apparatus 300 may be used for surgical procedures applied to other body regions or cavities, including, for example, the chest (for example, performing thoracoscopy to examine the pharynx and/or the larynx), etc.

The apparatus 300 includes a flexible body 304 passing through an inner channel of a rigid tube 302 of the apparatus 300. The rigid tube 302 provides an anchoring point and also enables better positioning and control of the flexible body 304 and the treatment tip 310. The one or more treatment tips, which may include, for example, a treatment tip with laser heated heating elements similar to the tips shown in FIGS. 1-4, is coupled to the distal end of the flexible tube 304. As shown, the treatment tip 310 is coupled to the flexible body 304 via a body element 312 having one end coupled to the treatment tip 310. The body element 312 may be, in some embodiments, a flexible chord or tube. The body member 312 may be secured, at its other end, to a distal end of the flexible body. Thus, under those circumstances, movement of the treatment tip 310 may be controlled, at least partially, by controlling and maneuvering the flexible body 304. In some embodiments, the body member 312 passes through a channel defined in the flexible body 304. Under those circumstances, the body member 312 may be extended (or advanced) through the opening at the distal end of the flexible body 304 to enable additional maneuverability and operability for the apparatus. The body member, which as noted may be a flexible tube or chord, can thus be further deployed from the position where the opening at the distal end of the flexible body 304 is located, and later retracted back into the flexible body 304.

Implemented in the arrangement of FIG. 5A is a tip maneuvering mechanism that may include mechanical and/or electrical actuation mechanisms. Such a maneuvering mechanism is configured to, for example, bend and tilt the flexible body 304 and/or the treatment tip 310. In some embodiments, movement of the treatment tip 310 may be controlled by controlling the movement of the flexible tube 304. Under those circumstances, movement of the scope (e.g., lateral pivoting of the tip of flexible tube endoscope, rotation of the tip, etc.) results in similar motion of the treatment tip. Thus, instead of having to implement independent treatment devices that each have their own actuation and control mechanisms, treatment tips that perform some treatment function can be coupled to an endoscope, and at least part of the operation of the treatment tip(s) can then be controlled through the normal actuation and control mechanisms of the scope-based apparatus. In addition, in some embodiments, actuation mechanisms that control movement only of the treatment tip, e.g., closing and opening of the closeable jaws, may be coupled to the treatment tip via the flexible body.

Another user controllable element is an energy activation button to activate the laser to irradiate the heating elements to thus cause the heating elements in the treatment tip 310 to be heated. Where the treatment tip 310 includes one or more heating elements, laser fibers to transmit laser radiation (generated, for example, by a laser device located outside the scope) may be passed through the working channel of the endoscope. In some embodiments, other types of radiation sources may be used.

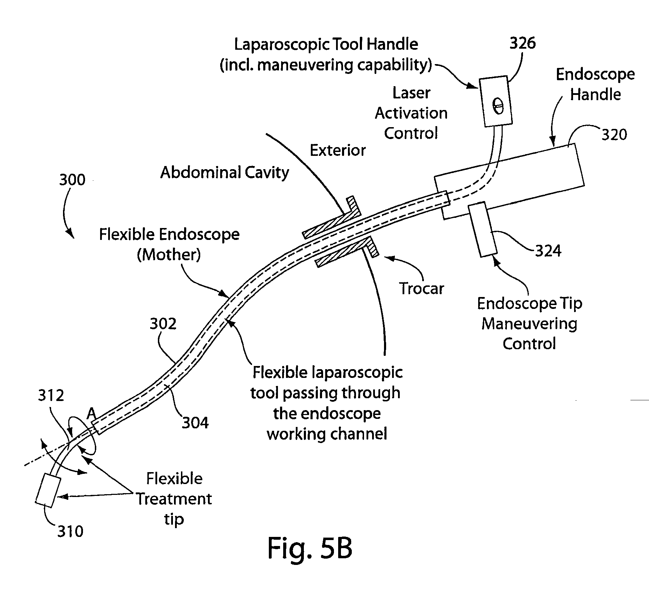

As noted, in some embodiments, a flexible body member coupled to the treatment tip may be passed through the inner (working) channel of the flexible body, and may be configured to be extended (or advanced) through the opening at the distal end of the flexible body. With reference to FIG. 5B, a schematic diagram of the scope-based apparatus 300 further including a flexible treatment tip with an extendible body member 314 is shown. The apparatus 300 includes the user controllable element 324 and a separate user controllable element 326. The controllable element 324 may be configured to only control the operation of the maneuverable tip, including controlling operations to extend the body member 312 (e.g., causing the flexible body member 312 to further advance from the distal end of the flexible body 304), controlling the spatial movement and position of the flexible tip and-controlling the operation associated with the implemented functionality of the tip (e.g., cutting or coagulating). The depicted controllable element 326 may be, on the other hand, configured to only control the operation of the flexible body 304, including controlling the spatial movement of the flexible body 304.

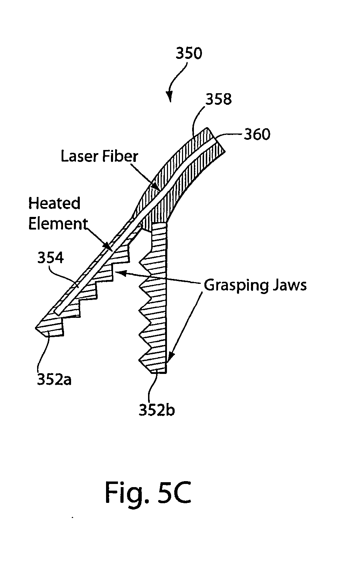

With reference to FIG. 5C, a schematic diagram of a treatment tip 350 to be coupled to a flexible body of a scope-based apparatus is shown. The treatment tip 350 includes two closeable jaws 352a and 352b that can be actuated to cause the jaws to close and open. The jaw 352a includes a heating element 354, which may be similar to the heating elements 114a, 114b, 214a and 214b depicted and described in relation to FIGS. 1-3. A laser fiber 360 is embedded through a base 358 of the treatment tip and has its distal end positioned proximate to the heating element 354. The base 358 may be coupled to a body member, such as body member 312 shown in FIGS. 5A and 5B, to secure the treatment tip 350 to the body member. The laser fiber extends through the flexible body (not shown) of the endoscope to which the treatment tip 350 is coupled to deliver laser radiation generated by a laser device (also not shown). Upon activation of the laser device, the laser radiation delivered via the fiber 360 irradiates the heating element to generate heat and raise the element's temperature relatively quickly.

Other types of treatment tools (e.g., blades, etc.) may similarly be coupled to the flexible body of the scope-based apparatus and manipulated (controlled) via the scope's actuation and control mechanisms.

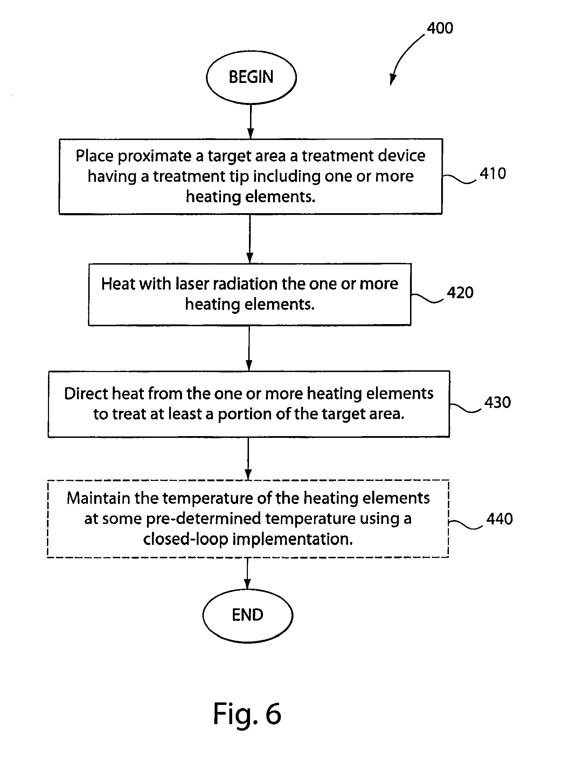

Referring to FIG. 6, a flow chart of a procedure 400 to treat a target area is shown. Initially, a treatment device that includes a treatment tip (and may include multiple treatment tips) is placed proximate 410 a target area to be treated. The treatment tip includes one or more members with respective contacting surfaces to contact the target area, e.g., grasping or touching the target area. In some embodiments, placing the treatment device proximate the target area is performed by securing the treatment tips to a flexible endoscope and passing a radiation waveguide (e.g., an optical fiber), as well as any treatment device control (e.g., actuation mechanisms to control operation of the treatment tips) through a working channel of the endoscope.

Having placed the treatment device proximate to the target area to be treated, one or more heating elements placed on at least one of the one or more of the members are heated 420. To heat the heating elements, laser radiation may be applied to the elements. As described herein, application of laser radiation to the heating elements can be performed by coupling radiation from a radiation source, such as, for example, a Nd:YAG or Ho:YAG laser, having 5-20 W of power, to an optical fiber(s) that passes along the body member coupled to the treatment tip and has its emitting end(s) positioned near the heating elements. Upon application of the laser radiation on the heating elements, the heating elements can reach high temperatures of, for example, 250º C, within a relatively short period of time, e.g., about 2 seconds.

Heat generated by the heating elements is directed 430 to at least a portion of the target area to be treated. In some embodiments, insulation layers may be lined along walls of cavities defined in the treatment tip's members in which the heating elements are disposed to prevent heat from being transferred to areas of the treatment tip other than the outer surface of the heating elements that come in contact with the target area. The heat directed to the target area may be applied to the target area (e.g., tissue of a patient) to coagulate blood vessels, ablate the tissue, cut the tissue, etc. As noted, in some embodiments, a treatment tip's member may include multiple heating elements provided on it to enable multiple concomitant application of heat to a patient's tissue being treated. For example, two heating elements arranged in a parallel orientation relative to each other may be included on a member such that two coagulation lines can be formed in the treated tissue, and the section of the tissue between the two coagulated lines can then be cut with minimal loss of blood.

Optionally, in some embodiments, the temperature of the heating elements is maintained 440 at some pre-determined temperature to ensure that the tissue treated and/or the treatment device are not overheated and/or otherwise damaged. Implementations to maintain the temperature at some pre-determined level include closed-loop implementations in which a temperature sensor is thermally coupled to at least one of the heating element of the treatment tip, and based on the measured temperature, the level of radiation applied to the heating elements is controlled or regulated.

A number of embodiments have been described. Nevertheless, it will be understood that various modifications may be made without departing from the spirit and scope of the invention. Accordingly, other embodiments are within the scope of the following claims.

Further aspects and optional features of the invention are set out in the following numbered clauses:
1. A treatment device comprising:
   a treatment tip including:
      one or more members with contacting surfaces to contact a target area, the tip being mechanically coupled to a body member; and
      one or more heating elements placed on at least one of the one or more members of the treatment tip, the one or more heating elements configured to be heated by laser radiation and to direct heat to treat at least a portion of the target area.
2. The treatment device of clause 1 further comprising:
   the body member
3. The device of clause 1 wherein the target area includes tissue of a patient.
4. The device of clause 1 further comprising at least one laser fiber having a distal end positioned proximate to the one or more heating elements, the at least one laser fiber configured to emit radiation substantially directed at the one or more heating elements.
5. The device of clause 1 further comprising a thermal insulation layer disposed proximate the one or more heating elements to thermally isolate the one or more heating elements from other areas of the treatment tip.
6. The device of clause 1 wherein the treatment tip includes at least one mechanically movable member.
7. The device of clause 6 wherein the at least one mechanically moveable member is rotateable about a longitudinal axis of the body member.
8. The device of clause 6 wherein the at least one mechanically moveable member includes at least one closeable jaw to hold a section of the target area, the at least one closeable jaw including at least one opposite facing contacting surface such that upon actuation of the at least one closeable jaw the at least one opposite facing contacting surface is displaced.
9. The device of clause 1 wherein the heating elements are configured to direct heat to the at least the portion of the area to be treated to cause one or more of: cutting a section of tissue, ablating the section of the tissue and coagulating a blood vessel within the section of the tissue.
10. The device of clause 1 further comprising:
   at least one sensor to measure temperature of at least one of the one or more heating elements; and
   a controller to regulate operations of the one or more heating elements based on the measured temperature of the at least one of the one or more heating elements.
11. The device of clause 1 wherein the one or more heating elements comprise:
   two heating elements arranged on one of the one or more members in a substantially parallel orientation such that application of heat to the at least the portion of the target area to be treated causes the formation of two coagulation sites on the target area.
12. The device of clause 11 further comprising a cutting instrument disposed between the two heating elements.
13. A treatment apparatus comprising:
   an endoscope containing a flexible body in a working channel of the endoscope; and
   a treatment tool having a treatment tip including one or more heating elements configured to be heated by laser radiation and to direct heat to treat at least a portion of a target area, the treatment tool coupled to a body member coupled to the flexible body.
14. The apparatus of clause 13 further comprising:
   at least one actuation mechanism to control movement of the treatment tool, at least a part of the at least one actuation mechanism disposed within the inner channel of the flexible body.
15. The apparatus of clause 13 wherein the body member comprises:
   an extendible body member passing through an inner channel of the flexible body.
16. The apparatus of clause 13 further comprising:
   at least one laser fiber having a distal end positioned proximate to the one or more heating elements, the laser fiber configured to emit radiation substantially directed at the one or more heating elements.
17. The apparatus of clause 14, wherein the treatment tip includes at least one closeable jaw to hold a section of the target area, the at least one closeable jaw including at least one
   opposite facing contacting surface such that upon actuation of the at least one closeable jaw the at least one opposite facing contacting surface is displaced.

## Claims

1. A device (200) comprising:
a first treatment tip (210) including a first member (240a) and a second member (240b) that define closeable jaws;
a body member (130) coupled to the first treatment tip (210);
at least two heating elements, including a first heating element (214a) and a second heating element (214b) disposed in cavities (116a) defined within in the first member (240a) and arranged substantially parallel relative to each other, the at least two heating elements configured to be heated by laser radiation emitted from at least one laser fiber.

2. The device of claim 1, further comprising:
a third heating element and a fourth heating element disposed in cavities (116b) defined within in the second member (240b) and arranged substantially parallel relative to each other.

3. The device of claim 1, further comprising:
a cutting tool (230) disposed between the first heating element (214a) and the second heating element (214b).

4. The device of claim 1, further comprising:
an actuation mechanism to control one or both of rotation and displacement of the closeable jaws.

5. The device of claim 1, further comprising:
at least one fiber (120a, 120b) having a distal end positioned proximate to the at least two heating elements , the at least one fiber configured to emit radiation substantially directed at the at least two heating elements.

6. The device of claim 1, further comprising:
thermal insulation material arranged to thermally isolate the at least two heating elements from other areas of the treatment tip (210).

7. The device of claim 1 further comprising:
a second treatment tip (110) comprising closable jaws, the second treatment tip (110) including:
one or more members (140a,140b) defining the closable jaws, the one or more members (140a,140b) having contacting surfaces (112a,112b), the second treatment tip (110) being mechanically coupled to the body member (130); and
at least a third heating element (114a, 114b), the third heating element configured to be heated by laser radiation.

8. The device of claim 7 further comprising:
at least one fiber (120a, 120b) having a distal end positioned proximate to the at least a thirds heating element (114a, 114b), the at least one fiber configured to emit radiation substantially directed at the at least a third heating element.

9. The device of claim 7 further comprising:
a cutting tool disposed between the first treatment tip (210) and the second treatment tip (110).

10. The device of claim 1 further comprising:
at least one sensor to measure temperature of the at least two heating elements; and
a controller to regulate operations of the at least two heating elements based on a measured temperature from the at least one sensor.
